# EUROPÄISCHE PATENTSCHRIFT

(11) **EP 1 956 982 B1**
(45) Veröffentlichungstag und Bekanntmachung des Hinweises auf die Patenterteilung: **07.01.2015**
(21) Anmeldenummer: 06806544.0
(22) Anmeldetag: 25.10.2006
(51) Int. Cl.: A61B 7/00

(54) **KÖRPERGERÄUSCH-FESTSTELLUNG**
DETECTION OF BODY SOUNDS
DETECTION DE BRUITS CORPORELS

(30) Priorität: 04.11.2005 DE 102005053109
(43) Veröffentlichungstag der Anmeldung: 20.08.2008
(73) Patentinhaber: Heinen & Löwenstein GmbH & Co. KG, 56130 Bad Ems (DE)
(72) Erfinder: KOEHLER, Ulrich, 35043 Marburg (DE); GROSS, Volker, 34435 Wettenberg (DE)
(74) Vertreter: Tergau & Walkenhorst
(86) Internationale Anmeldenummer: PCT/EP2006/010295
(87) Internationale Veröffentlichungsnummer: WO 2007/051556

(56) Entgegenhaltungen:
- EP-A- 0 951 867
- EP-A- 1 495 721
- WO-A-2004/002317
- US-A1- 2002 013 538
- US-A1- 2004 225 226
- US-A1- 2005 165 323
- HOMS-CORBERA, A.; JANE, R.; FIZ, J.A.; MORERA, J.;: "Algorithm for time-frequency detection and analysis of wheezes" PROCEEDINGS OF THE 22ND ANNUAL INTERNATIONAL CONFERENCE OF THE IEEE ENGINEERING IN MEDICINE AND BIOLOGY SOCIETY, 2000., Bd. 4, 23. Juli 2000 (2000-07-23), Seiten 2977-2980, XP002459683

## Beschreibung

Die Erfindung betrifft ein Verfahren und eine Vorrichtung zum Feststellen und Überwachen von Körpergeräuschen bei Mensch und Tier gemäß dem Oberbegriff der Ansprüche 1 und 7.

Weithin bekannt ist das zum Abhören von Körpergeräuschen übliche Stethoskop, bei dem von einer Schallaufnehmerkapsel Schlauchleitungen zu Ohrhörern führen. Man beobachtet damit Schallerscheinungen an Herz, Lungen, Unterleib, Schilddrüse usw., um Auffälligkeiten festzustellen und zu diagnostizieren.

Mit akustischen Wandlern sind Identifikations-Signale erzeugbar, die eine Analyse ermöglichen. Beispielsweise beschreibt US 5 259 373 eine Beatmungsvorrichtung, mit der Druckschwankungen registriert werden können. Gemäß DE 101 59 384 wird im Gaszufuhrsystem ein Schallaufnehmer angeordnet, dessen Signale Rückschlüsse auf den Lungenzustand zulassen. Zur Auswertung erfolgt ein Vergleich mit bekannten Klang- bzw. Geräuschbildern, etwa um einen Sekretstau zu erkennen.

EP 0 504 945 A2 offenbart eine mobile Vorrichtung, die mittels Lageaufnehmer, Elektroden, Kehlkopfmikrofon und Fingersensor unter Beachtung der Körperlage das Erfassen von Herzfrequenz, Atmungslauten und Blut-Sauerstoffsättigung erlaubt. Die gespeicherten Daten werden mit einem Rechner analysiert, um Vorhandensein und Ausprägung eines Schlaf-Apnoe-Syndroms zu ermitteln. Weitergehende Untersuchungen sind nicht vorgesehen bzw. mit Unsicherheiten verbunden.

Zur Aufzeichnung und Analyse vor allem von Atmungsgeräuschen bei Säugetieren einschließlich Menschen wurde in US 6 261 238 B1 ein sog. Phonopneumograph-System vorgeschlagen. Es erfordert großen Aufwand und benötigt zur Erkennung und Analyse diverser Geräuscharten eine ganze Anzahl von Atmungssensoren und nachgeordneten

### BESTÄTIGUNGSKOPIE

Einrichtungen für die Signalübertragung und -verstärkung einschließlich Digitalisierung zwecks Verarbeitung in einem Rechner mit Monitor und Drucker. Ferner sind Speicher- und Abhöreinrichtungen vorgesehen. Das System setzt eine Erkennung der Atemphasen voraus und bedingt eine Vielzahl von Spektralkurven-Anpassungen mit Einzelberechnung jeder Kurve, d.h. zahlreiche serielle Schritte mit hoher Rechenintensität zur Meßwert-Erfassung und -Auswertung. Bei nächtlicher Überwachung während 8 Stunden sind z.B. 8 x 60 x 60 x 20 = 576.000 Einzelberechnungen notwendig. Die erste Einstufung kann die Genauigkeit der folgenden Schritte stark beeinflussen oder stören. Wird ein Geräusch anfangs fälschlich als Hintergrundgeräusch eingestuft, so unterbleibt eine weitergehende Analyse.

EP 0 951 867 A2 offenbart ein Verfahren gemäß dem Oberbegriff des Anspruchs 1.

Die herkömmlichen Systeme haben ferner oft den Nachteil, daß relativ viele Sensoren benötigt werden, z.B. sechs akustische Sensoren, über deren räumliche Anordnung bzw. Verteilung an einem Objektkörper nur unzureichende Angaben gemacht werden.

Es ist ein wichtiges Ziel der Erfindung, das Beobachten, Aufzeichnen und Analysieren von Körpergeräuschen bei Mensch und Tier erheblich zu vereinfachen. Mit wirtschaftlichen Mitteln soll es möglich sein, das Auftreten von Anomalien ohne örtliche und tageszeitliche Beschränkungen zuverlässig zu erkennen und zu beurteilen, wobei Langzeitmessungen und -aufzeichnungen von Atemgeräuschen vor allem auch nachts durchführbar sein sollen. Angestrebt wird ferner, eine einfach zu bedienende und vielseitige mobile Gerätschaft zu entwickeln. Hauptmerkmale der Erfindung sind in den Ansprüchen 1 und 7 angegeben.

Ausgestaltungen sind Gegenstand der Ansprüche 2 bis 6 sowie 8 bis 13.

Ein Verfahren zum Feststellen und Überwachen von Körpergeräuschen bei Mensch und Tier unter Verwendung von bioakustischen Sensoren und nachgeordneten Analysatoren zeichnet sich erfindungsgemäß laut Anspruch 1 aus.

Damit ist es im Unterschied zum Stand der Technik möglich, z. B. den Atmungsverlauf eines Probanden nichtinvasiv nicht nur tagsüber, sondern vor allem nachts und in Zeiträumen von vielen Stunden auch mobil zu überwachen. Das ist ohne Weiteres auch bei Kleinkindern und Säugetieren durchführbar, die keinen Anweisungen folgen können. Die Langzeit-Meßergebnisse und Kurvenvergleiche liefern außerdem aussagefähige oder zumindest eingrenzbare Ist-Werte und Muster, aus denen sich verläßlich krankheitsspezifische Symptome objektivieren lassen. Dazu ist es nicht (wie herkömmlich) notwendig, ein Geräusch vorab positiv als echtes oder reines Geräusch eines Säugetier-Atmungssystems zu identifizieren. Auch genügt es zur Detektion lautstärkeabhängiger Charakteristika, lediglich einen Vergleich der Amplituden einzelner Geräuschkanäle heranzuziehen. Geräusche außerhalb des jeweils interessierenden Frequenzbereiches werden z.B. mittels eines analogen Bandpaß-Filters eliminiert.

Die einzelnen Transformations-Zeitfenster wählt man z.B. für die Analyse von Atemgeräuschen vorteilhaft so, daß sich Auflösungen von ca. 0,1 s ergeben. Zur Gewinnung frequenzspezifischer Charakteristiken ist es günstig, eine Fast-Fourier-Transformation der Daten in den Spektralbereich vorzunehmen, wobei pro Transformations-Zeitfenster eine Spektralkurve entsteht, mit der Amplituden über der Frequenz dargestellt und aufgezeichnet werden.

Gemäß einer Weiterbildung werden aus den Spektralkurven jeweils wenigstens zwei Frequenzbereiche ausgewählt und Medianwerte der Amplituden bestimmt, die Stützwerten einer Anpassungslinie entsprechen, worauf zugeordnete Anpassungslinien auf der Basis von Stützstellen-Werten entwickelt werden, namentlich durch deren lineare oder quasilineare Verbindung. In einem X,Y-Koordinatensystem sind die Stützwerte Y-Beträge bei einer Frequenz X, die der zeitlichen Mitte des jeweiligen Frequenzbereiches entspricht.

Zur Verfeinerung des Verfahrens kann man wenigstens einen dritten, vorzugsweise mittleren Frequenzbereich auswählen und damit weitere Stützstellen für genauere Anpassungslinien einführen. Auf diese Weise wird eine sehr gute Grundlage für die fortlaufende Kontrolle und Auswertung gewonnen, ohne daß ein übergroßer Rechenaufwand betrieben werden müßte.

Zur Suche nach lokalen Maxima vergleicht man die Y-Werte der Spektralkurve mit den Y-Werten der Anpassungslinie unter Ansatz eines Schwellenwertkriteriums. Die Identifikation erfolgt, falls das lokale Maximum an der Stelle X einen Schwellenwert derart übersteigt, daß der Y-Wert der Spektralkurve größer ist als die Summe des Y-Werts der Anpassungslinie und einer Grundkonstante K1. Deren Vorgabe erfolgt nach Erfahrungswerten aus statistisch gesicherten Meßreihen.

Besonders günstig ist es, wenn an Stelle nur der Grundkonstante K1 ein Term bestehend aus einer Anteilskonstante K2 und dem Medianwert der Abweichungen der Spektralkurve von der Anpassungslinie multipliziert mit einem Dynamikfaktor hinzuaddiert wird, so daß nun der Y-Wert der Spektralkurve größer ist als die Summe des Y-Werts der Anpassungslinie und der Anteilkonstante K2 sowie einer Dynamikkonstante K3 multipliziert mit dem Median der Abweichungen der Spektralkurve von der Anpassungslinie.

Für die Analyse von Wheezing beim Menschen wählt man in vorteilhafter Verfahrensgestaltung an den Enden eines Bereichs von 150 Hz bis 1.600 Hz zwei Frequenz-Teilbereiche aus, deren Umfang durch einen Faktor eines vorbestimmbaren Peakkriteriums zur Auswahl der lokalen Maxima gegeben ist, z. B. einer Frequenzbreite von 100 Hz. Dieser Faktor kann zum Beispiel 1 oder 2 sein. Zur noch besseren Anpassung des Verfahrens an den Menschen und dessen Atemgeräusche wird in einem dritten Frequenzbereich innerhalb der Spektralkurve eine weitere Stützstelle gebildet, z.B. bei ca. 700 Hz für Atemgeräusche Erwachsener; bei Kindern wählt man eine etwas höhere Frequenz.

In einem bevorzugten Ausführungsbeispiel, welches signifikante Wheezing-Phänomene zuverlässig identifiziert, schließt sich gemäß Anspruch 5 an die Suche nach lokalen Maxima noch die Anwendung von Plausibilitätskriterien an, die auf gesicherten Meßergebnissen beruhen, insbesondere wie folgt:
i) das lokale Maximum im Frequenzbereich innerhalb eines Transformations-zeitfensters ist beim Überschreiten des Schwellenwertkriteriums nicht breiter als 100 Hz;
ii) die gefundenen Maxima liegen in einem Frequenzband zwischen 150 Hz und 1600 Hz;
iii) die Frequenzveränderung des stärksten der lokalen Maxima (dominante Frequenz) beträgt zwischen zwei aufeinanderfolgenden Transformations-Zeitfenstern weniger als 100 Hz;
iv) die Wheezing-Ereignisse haben eine Mindestlänge größer als 0,3 s, d.h. sie treten bei einer zeitlichen Auflösung von 0,1 s in mindestens vier aufeinanderfolgenden Transformations-Zeitfenstern auf;
v) die Wheezing-Ereignisse dauern nicht länger als die doppelte Zeit einer normalen mittleren Exspirationsphase der jeweiligen Säugetiergattung (die bei Menschen ca. 4 s beträgt).

Anschließend kann für jedes Diagnose-Zeitsegment, z.B. 30 s oder ein kompletter Atemzug, eine zusammenfassende Wheezing-Rate gewonnen werden, indem erkanntes Wheezing aller Körpersensoren zusammengefaßt wird, wobei sich die Rate auf den zeitlichen Anteil aller Ereignisse bezieht, die von mindestens einem Sensor erkannt wurden. Tritt ein lautes Signal zeitgleich nur im Außenkanal auf, so handelt es sich mit großer Wahrscheinlichkeit um ein Außengeräusch und das Signal wird als Artefakt verworfen.

Eine Vorrichtung zum Feststellen und Überwachen von Körpergeräuschen bei Mensch und Tier unter Verwendung von bioakustischen Sensoren und nachgeordneten Analysatoren, insbesondere zum Durchführen des Verfahrens nach einem der Ansprüche 1 bis 6, offenbart Anspruch 7.

Das System ist also insgesamt einfach und mithin kostengünstig, aber effektiv. Es läßt sich mobil verwenden und ist sehr praktisch, weil die Handhabung keine Probleme bereitet.

Es wurde überraschend festgestellt, daß für die Überwachung der Atemgeräusche maximal drei akustische Körper-Sensoren genügen, die man nahe der Luftröhre und jeweils im Bereich eines Lungenflügels anbringt, bevorzugt etwa mittig über jedem Lungenflügel bzw. unterhalb des Schulterblattes und (beim Menschen) im Interkostalraum zwischen der 5. und 7. Rippe. So ist der apparative Aufwand gegenüber herkömmlichen Vorrichtungen stark verringert und zugleich die Zuverlässigkeit der Benutzung gesteigert. Damit man Geräusche erfassen und ausschließen kann, welche für die Untersuchung unerheblich sind, wird zweckmäßig zusätzlich ein Umgebungssensor vorgesehen, der z.B. an einer Sensorkabelbox oder an der Rückseite eines trachealen Sensors anbringbar ist. Zusätzlich kann ein Sennsor zum Abtasten von Brustkorbbewegungen, insbesondere zum Erfassen der Atemaktiivität vorhanden sein.

Die Vorrichtung erfordert also zum Aufnehmen und Übertragen der Geräuschsignale bzw. -daten insgesamt nur bis zu vier separierbare bzw. separierte Kanäle, deren Aufzeichnungen automatisch und/oder audiovisuell auswert- und analysierbar sind. An oder in den bioakustischen Sensoren oder in Wirkverbindung mit ihnen sind allgemein Analog-/Digital-Konverter vorgesehen, deren Ausgang digitale Signale bzw. Signalfolgen liefert. Diese werden mit wenigstens einem Übertrager weitergeleitet, der mit einem Datenspeicher und/oder einer Transfereinrichtung gekoppelt ist.

Zum relativ bequemen Tragen der bioakustischen Sensoren kann man vorteilhaft ein Halsband und/oder einen Brustgurt o.dgl. verwenden, wobei Schulterträger und/oder Klettverschlüsse vorhanden sein können. Als akustische Sensoren eignen sich vor allem luftgekoppelte oder Piezo-Mikrofone, die man am Objektkörper z.B. mit Klebefolie oder Pflaster an ausgesuchten Stellen fixieren kann, die aber auch in einen Brustgurt eingearbeitet sein können.

In an sich bekannter Weise läßt sich die Grundausstattung ergänzen, etwa durch einen Lagesensor, einen Bewegungssensor für den Brustkorb und/oder einen Sensor für den Sauerstoffgehalt im Blut, den man z.B. an einem Ohr oder an einer Extremität anbringt. Ferner kann man zur Erhöhung der Empfindlichkeit und zur Sicherheit gegen Störungen Vorverstärker und Analog-Filter vorsehen, speziell Bandpaßfilter.

Solche oder sämtliche Hilfsmittel lassen sich in einem kleinen Gehäuse unterbringen. Günstig ist die Gestaltung als am Körper tragbarer oder auch integrierter (Signal-) Datenlogger, der kein gesondertes Gerät sein muß, sondern auch zu einer Analysestation oder einem Schlafzentrum gehören kann und zur Überwachung von Körpergeräuschen und Aufzeichnung der Signale von maximal vier Geräuschkanälen bevorzugt mit akustischen Mikrospeichern ausgestattet ist, z.B. mit zwei Stereo-Mini-Einheiten in der Art von MP3-Recordern.

Weitere Merkmale, Einzelheiten und Vorteile der Erfindung ergeben sich aus dem Wortlaut der Ansprüche sowie aus der folgenden Beschreibung von Ausführungsbeispielen anhand der Zeichnungen. Darin zeigen:
- Fig. 1: ein Schema einer Meßkette,
- Fig. 2: ein Block-Fließschema des erfindungsgemäßen Verfahrens,
- Fig. 3: eine typische Spektralkurve eines Transformations-Zeitfensters.
- Fig. 4: eine Spektralkurve ähnlich Fig. 3, jedoch mit lokalen Maxima,
- Fig. 5: eine Spektralkurve ähnlich Fig. 4, jedoch mit einer Anpassungslinie und mit Kurven-Stützstellen,
- Fig. 6: einen Stützstellen-Kurvenausschnitt mit Median- und Mittelwert,
- Fig. 7: eine schematisierte Darstellung der Tragweise eines Datenloggers,
- Fig. 8: eine Schrägansicht einer Tragvorrichtung und
- Fig. 9: eine Ausschnittvergrößerung zu Fig. 8.

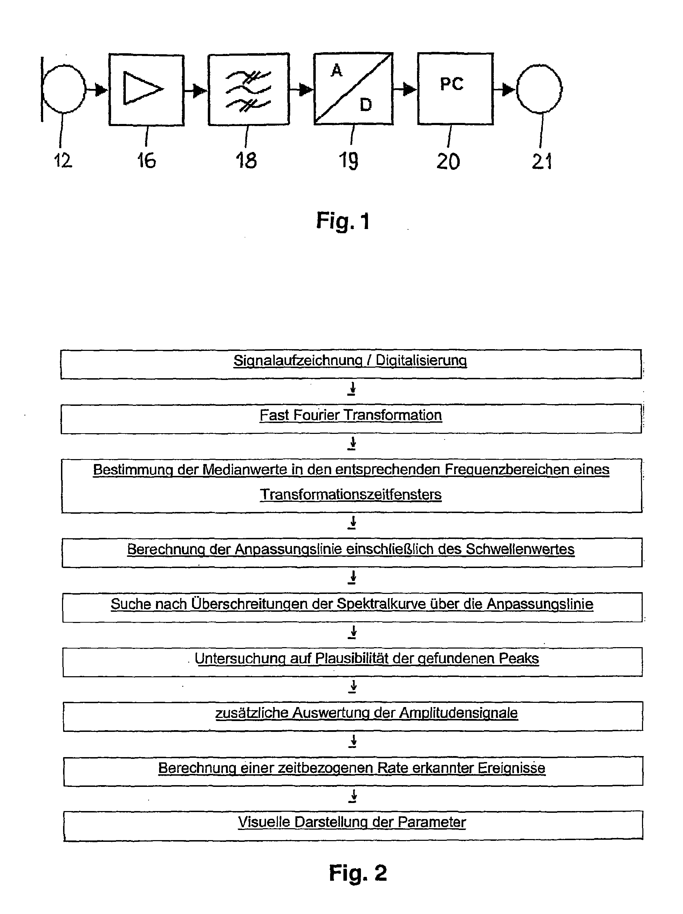

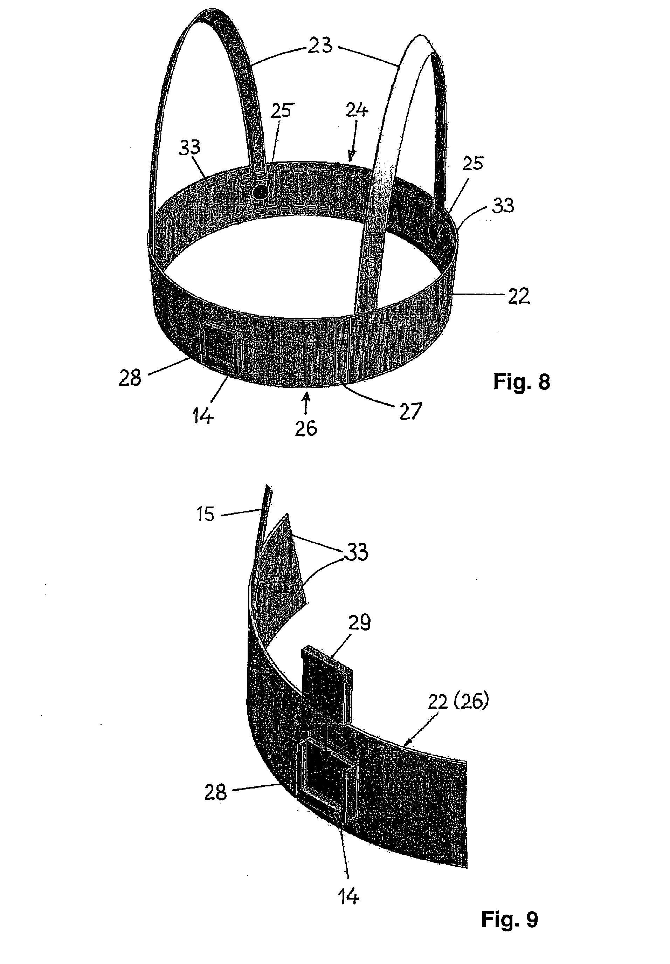

Eine erfindungsgemäße Vorrichtung zum Feststellen und Überwachen von Körpergeräuschen bei Mensch und Tier weist nur wenige Komponenten auf, nämlich bioakustische Sensoren und nachgeordnete Analyse-Hilfsmittel, die sich insgesamt in einem kleinen Gehäuse unterbringen lassen. Die daher mobil einsetzbare, allgemein mit 10 bezeichnete und auch Datenlogger genannte Vorrichtung benötigt maximal drei akustische Sensoren 12, die z.B. mittels Klebefolien an ausgewählten Körperstellen anbringbar sind. Den Aufbau einer typischen Meßkette zeigt Fig. 1. Ein Sensor 12 gibt Signale an den Verstärker 16 ab, dem die bevorzugt als Bandpaßfilter ausgebildete Filtereinheit 18 nachgeordnet ist. Deren Ausgangssignale werden in wenigstens einem Analog/Digital-Konverter 19 digitalisiert und in dieser Form der Speicher-/Rechner-einheit 20 (z.B. Speicherkarten, deren Daten anschließend in einem Rechner ausgewertet werden) zugeführt. Zusätzlich kann ein Umgebungssensor 14 vorhanden und mittels Kabeln 15 (Fig. 9) mit einer Box verbunden sein, in der sich der Verstärker 16 sowie das nachgeschaltete Filter 18 und der A/D-Konverter 19 befinden können. Der weitere Hauptbestandteil in Form des Speichermediums 20 kann sich direkt in dem Gehäuse befinden oder per Kabel oder Funk mit ihm verbunden sein.

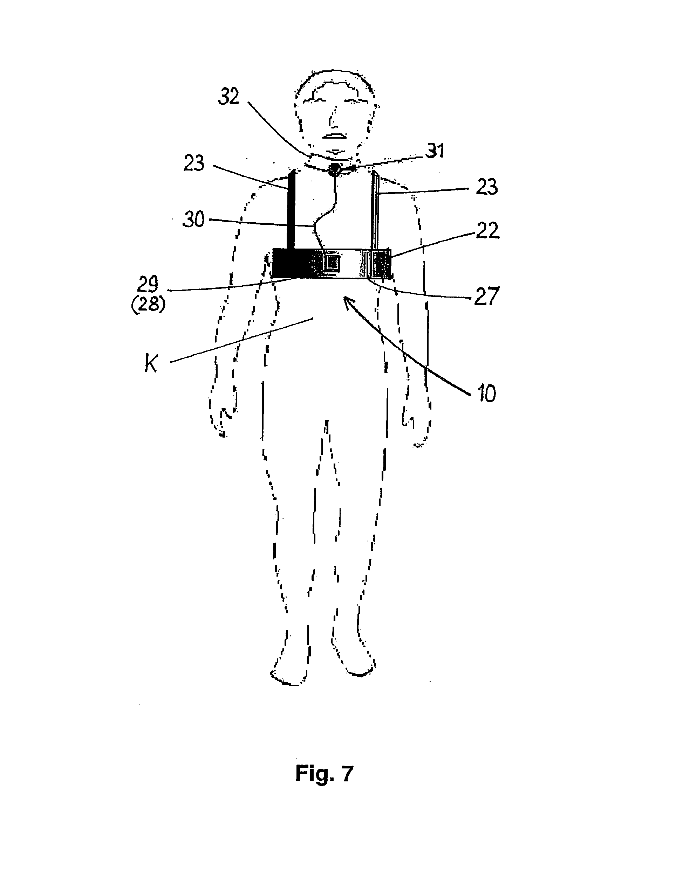

Die drei akustischen Sensoren 12, die luftgekoppelte oder Piezo-Mikrofone sein können, werden z.B. mittels der Klebefolien am Objektkörper K (Fig. 7) angebracht, und zwar einer (31) im Bereich der Luftröhre und je einer (25 in Fig. 8) im Bereich der Lungenflügel, bevorzugt etwa mittig über jedem Lungenflügel bzw. unterhalb des Schulterblattes und beim Menschen im Interkostalraum zwischen 5. und 7. Rippe. Der nahe der Trachea angeordnete Sensor 12 bzw. 31 kann auf seiner Rückseite den Umgebungssensor 14 tragen, z.B. an einem gemeinsamen Halsband 32 (vergl. Fig. 7). Die Halterung am Oberkörper K unterstützt vorteilhaft ein Brustgurt 22, der - insofern ähnlich einem Büstenhalter - Schulterträger 23 aufweisen und zur Gewichtsentlastung für die Sensoren 12, 14 samt ihren Kabeln 15 beitragen kann. Eventuell ist zusätzlich ein Bewegungssensor 33 für den Brustkorb und/oder ein Lagesensor vorhanden, gegebenenfalls ferner ein etwa am Ohr oder an einer Extremität anzubringender Blut-O₂-Sensor.

Der Verstärker 16 kann in Gehäusen der Sensoren 12, 14 angeordnet sein oder in Wirkverbindung mit ihnen stehen. Dem A/D-Konverter 19 folgt eine Transfereinrichtung z.B. in Form eines Leitungsstrangs oder einer Funkstrecke, die auf kürzerem oder längerem Weg zu dem Datenspeicher oder der Auswerteeinheit 20 führt, weil diese sich nicht im gleichen Raum befinden muß; sie kann je nach gegebenen örtlichen Verhältnissen nah oder fern angeordnet sein. Das System ist mit Kommunikationsnetzen verbindbar und wird durch wenigstens einen (nicht dargestellten) Energieversorger komplettiert, der in an sich bekannter Weise aus einem Netzteil oder einem Akku- oder Batterieblock bestehen kann.

Aus Fig. 2 gehen die typischen Schritte des Verfahrens nach der Erfindung hervor. Sie lassen sich in dieser oder ähnlicher Sequenz auf verhältnismäßig einfache und übersichtliche Weise programmieren.

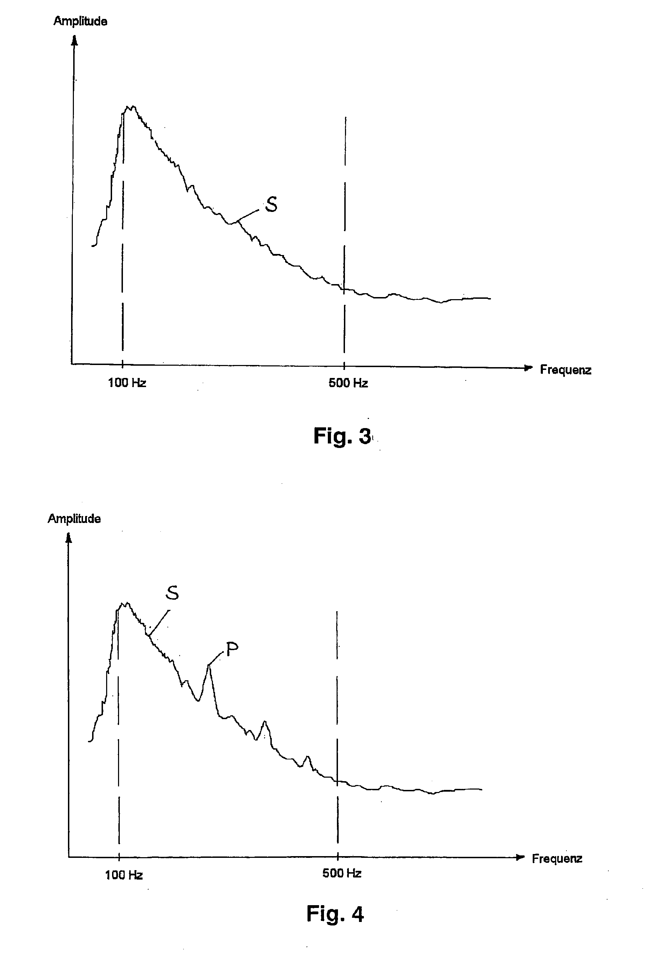

In Fig. 3 ist eine typische Spektralkurve S eines Transformations-Zeitfensters während der Atmung eines erwachsenen Menschen dargestellt, wobei die relative Signalamplitude über der Frequenz aufgetragen ist. Man erkennt deutlich, daß ein Maximum bei oder knapp über etwa 100 Hz liegt, wogegen höherfrequente Geräusche ab etwa 500 Hz zu einem Niedrigwert hin abklingen.

Eine vergleichbare Spektralkurve S geht aus Fig. 4 hervor, wobei die Atmung eines Erwachsenen in einem mittleren Bereich - hier zwischen etwa 200 Hz und 500 Hz-lokale Maxima zeigt. Einem Scheitelwert oder Hauptpeak P entspricht ein Wheezing-Ereignis; harmonische Oberwellen lassen abgeschwächte Scheitelwerte erkennen.

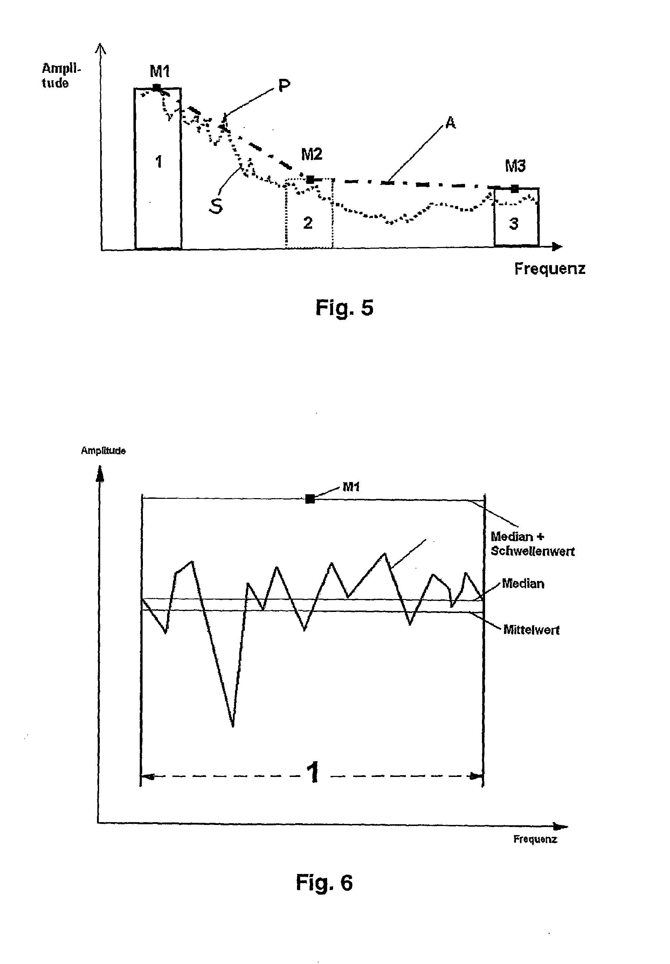

Die erfindungsgemäße Auswertung benutzt medianbasierte Stützstellen, wie in Fig. 5 und 6 gezeigt. Eine Spektralkurve S hat im Beispiel einer normalen Atmung eines Erwachsenen mit Wheezing einen Hauptpeak P zwischen zwei Kurven-Stützstellen M₁ und M₂. Eine berechnete Anpassungslinie A, zu der ein Schwellenwert bereits addiert ist, liegt allgemein oberhalb der Spektralkurve S, die aber mit dem Hauptpeak P die Anpassungslinie A (inklusive Schwellenwert) überschreitet.

Fig. 6 stellt schematisch dar, wie ein Ausschnitt einer Spektralkurve in einem ausgewählten unteren Frequenzbereich "1" zur Gewinnung eines Stützstellenwerts M₁ herangezogen wird. Dieser ist die Summe aus einem Schwellenwert und dem Medianwert der Spektralkurve S, welcher im gezeigten Beispiel oberhalb des Mittelwerts liegt und sie generell besser als letzterer charakterisiert, weil die Amplituden in aller Regel nicht symmetrisch zum Mittelwert verteilt sind, also keiner Gaußverteilung unterliegen. - Die Stützstellenwerte M₂ und M₃ werden entsprechend M₁ errechnet. Für eine gröbere Anpassung können ein frequenzmäßig unterer und ein oberer Stützstellenwert M₁, M₃ genügen, so daß die Berechnung der mittleren Stützstelle M₂ entfallen kann.

In Fig. 7 ist schematisiert gezeichnet, wie am Körper K eines Erwachsenen ein Datenlogger 10 angebracht wird. Wie Fig. 8 und 9 deutlicher zeigen, dient dazu ein Brustgurt 22, der mit Schulter-Trägern 23 versehen ist und in einem Rückenteil 24 zwei Mikrofone 25 aufweist. Am Vorderteil 26 des Brustgürtels 22, der mit einem Klettverschluß 27 geschlossen und geöffnet werden kann, befindet sich eine Halterung oder Aufnahme 28 für eine abnehmbare Datenbox 29, zu der eine Zuleitung 30 von einem Trachealmikrofon 31 führt. Am Innenumfang des Brustgürtels 22 ist wenigstens ein Atmungssensor 33 eingearbeitet. Beispielsweise an der Aufnahme 28 kann ferner ein Außenmikrofon als Umgebungssensor 14 angebracht sein.

Die Erfindung ist nicht auf eine der vorbeschriebenen Ausführungsformen beschränkt, sondern in vielfältiger Weise abwandelbar. Man erkennt jedoch, daß sie mit insgesamt niedrigem Aufwand eine - stationäre oder mobile - Langzeitüberwachung z.B. der Atmung von Patienten ermöglicht, so daß deren Lungengeräusche festgestellt und mit ihren Meßdaten gespeichert werden, die vor allem zur Früherkennung von Erkrankungen und akuten Störungen unmittelbar zur Verfügung stehen. Ähnliches gilt für die Überwachung von Darmgeräuschen, die zur Beurteilung der Peristaltik, z.B. für die Früherkennung eines mechanischen/paralytischen Ileus sehr wichtig ist. Im klinischen Bereich schafft die Erfindung ein Frühwarnsystem mit sofortiger Signalgebung, die ohne das Erfordernis einer Bedienung von Einzelgeräten Ärzte und Pflegepersonal in die Lage versetzt, im Notfall schnellstens zu handeln. Die Vorrichtung benötigt maximal drei bioakustische Sensoren (12), die jeweils an einer dem Untersuchungsbereich zugewandten Stelle eines Objektkörpers (K) fixierbar sind, maximal einen Sensor (14) zum Aufzeichnen von Umgebungsgeräuschen, maximal vier separierbare Kanäle (11) zum Aufnehmen und Übertragen von durch die Sensoren (12) erfaßten Geräuschsignalen bzw. -daten und Einrichtungen zur Energieversorgung sowie zum Weiterleiten, Umwandeln, Speichern und Darstellen von Signal- bzw. Datenfolgen an oder in einen Recorder oder eine Recheneinheit (20). Der Datenlogger (10) ist mit einem Brustgurt (22) kombinierbar, der an einem Objektkörper (K) anleg- und abnehmbar ist, zur Gewichtsentlastung Schulterträger (23) und insbesondere rückseitig zwei bioakustische Sensoren (14; 25) sowie vorderseitig eine Aufnahme (28) für eine Datenbox (29) aufweist, die über eine Zuleitung (30) mit einem Trachealmikrofon (31) verbunden ist.

Sämtliche aus den Ansprüchen, der Beschreibung und der Zeichnung hervorgehenden Merkmale und Vorteile, einschließlich konstruktiver Einzelheiten, räumlicher Anordnungen und Verfahrensschritten, können sowohl für sich als auch in den verschiedensten Kombinationen erfindungswesentlich sein.

**Bezugszeichenliste**

| | | | |
|---|---|---|---|
| A | | Anpassungslinie | |
| M₁ M₂ M₃ | | medianbasierte Stützstellen | |
| P | | Peak | |
| S | | Spektralkurve | |
| 1, 2, 3 | | Frequenzbereiche | |
| 10 | Gerät / Datenlogger | 23 | Träger |
| 12 | akustische Sensoren | 24 | Rückenteil |
| 14 | Umgebungssensor | 25 | (Rücken-)Mikrofone |
| 15 | Kabel | 26 | Vorderteil |
| 16 | Verstärkerbox | 27 | Klettverschluß |
| 18 | Filterbox | 28 | Aufnahme / Datenboxhalter |
| 19 | A/D-Konverter | 29 | Datenbox |
| 20 | Rechner(einheit) / Laptop | 30 | Zuleitung(en) |
| 21 | Anzeige / Bildschirm | 31 | Trachealmikrofon |
| 22 | Brustgurt | 32 | Halsband |

## Patentansprüche

1. Verfahren zum Feststellen und Überwachen von Körpergeräuschen bei Mensch und Tier unter Verwendung von bioakustischen Sensoren und nachgeordneten Analysatoren, wobei bioakustische Sensoren an maximal drei ausgewählten Stellen eines Objektkörpers angebracht werden, dass maximal ein zusätzlicher bioakustischer Sensor zur Aufzeichnung von Außengeräuschen vorhanden ist, dass mit den Sensoren innerhalb eines wählbaren Zeitsegments fortlaufend Geräuschsignale und/oder -daten erfasst werden, dass die so erzeugten Folgen von Geräuschsignalen und/oder -daten digitalisiert, gespeichert und in frequenzabhängige Spektralkurven transformiert werden, **dadurch gekennzeichnet, dass** aus den Spektralkurven fortlaufend zugeordnet jeweils wenigstens zwei Frequenzbereiche ausgewählt und Medianwerte der Amplituden bestimmt werden, die als Stützwerte für Anpassungslinien bereitgestellt werden, wobei zugeordnete Anpassungslinien durch lineare oder quasilineare Verbindung der Stützstellen-Werte entwickelt werden, und dass ein Vergleich der Anpassungslinien mit den entsprechenden Spektralkurven zur auswert- und signalisierbaren Erkennung spezifischer Geräuschsymptome erfolgt, z. B. Wheezing, Ronchus, Husten o. dgl.

2. Verfahren nach Anspruch 1, **gekennzeichnet durch** eine solche Wahl der Transformations-Zeitfenster, dass sich Auflösungen von ca. 0,1s ergeben.

3. Verfahren nach einem der Ansprüche 1 oder 2, **dadurch gekennzeichnet, dass** eine Fourier-Transformation oder eine Fast-Fourier-Transformation der Daten in einen Spektralbereich vorgenommen wird, wobei pro Transformations-Zeitfenster eine Spektralkurve entsteht, mit der Amplituden über der Frequenz dargestellt, übertragen und aufgezeichnet werden.

4. Verfahren nach einem der Ansprüche 1 bis 3, **dadurch gekennzeichnet, dass** die Stützwerte im X,Y-Koordinatensystem jeweils Y-Beträge an einer Frequenzstelle X sind, und dass die Auswertung durch Vergleich der Y-Werte von Spektralkurve und Anpassungslinie anhand von Schwellenwerten erfolgt.

5. Verfahren zum Feststellen und Überwachen von Atemgeräuschen bei Mensch und Tier unter Verwendung von bioakustischen Sensoren und nachgeordneten Analysatoren, insbesondere nach einem der Ansprüche 1 bis 4, **dadurch gekennzeichnet, dass** man zur Wheezing-Detektion anhand gesicherter Meßergebnisse Plausibilitätskriterien anwendet, nämlich:
i) Das lokale Maximum im Frequenzbereich innerhalb eines Transformationszeitfensters ist beim Überschreiten des Schwellenwerts nicht breiter als 100 Hz;
ii) Die gefundenen Maxima liegen in einem Frequenzband zwischen 150 Hz und 1600 Hz;
iii) Die Frequenzänderung des stärksten der lokalen Maxima (dominante Frequenz) beträgt zwischen zwei aufeinanderfolgenden Transformations-Zeitfenstern weniger als 100 Hz;
iv) Die Wheezing-Ereignisse haben eine Mindestdauer von 0,3s, d. h. sie treten bei einer zeitlichen Auflösung von 0,1s in mindestens vier aufeinanderfolgenden Transformations-Zeitfenstern auf;
v) Die Wheezing-Ereignisse dauern nicht länger als die doppelte Zeit einer normalen mittleren Exspirationsphase der jeweiligen Säugetiergattung (d. h. bei Menschen < 2x ca. 4s).

6. Verfahren zum Feststellen und Überwachen von Atemgeräuschen bei Mensch und Tier unter Verwendung von bioakustischen Sensoren und nachgeordneten Analysatoren, insbesondere nach einem der Ansprüche 1 bis 5, **dadurch gekennzeichnet, dass** man zur Hustendetektion das Auftreten steiler Flanken des Außengeräusch-Amplitudensignals unter der Voraussetzung auswertet, dass zugleich große Amplituden oder Übersteuerungen bei allen anderen dem Objektkörper zugewandten Signalen festzustellen sind.

7. Vorrichtung zum Feststellen und Überwachen von Körpergeräuschen bei Mensch und Tier unter Verwendung von bioakustischen Sensoren (12) und nachgeordneten Analysatoren, denen Daten- bzw. Signalfolgen zugeführt werden, insbesondere zum Durchführen des Verfahrens nach einem der Ansprüche 1 bis 6, **gekennzeichnet durch** folgende Komponenten:
• maximal drei bioakustische Sensoren (12), die jeweils an einer dem Untersuchungsbereich zugewandten Stelle eines Objektkörpers (K) fixierbar sind,
• maximal einen Sensor (14) zum Aufzeichnen von Umgebungsgeräuschen,
• maximal vier separierbare Kanäle (11) zum Aufnehmen und Übertragen von **durch** die Sensoren (12) erfassten Geräuschsignalen bzw. -daten und
• Einrichtungen zur Energieversorgung sowie zum Weiterleiten, Umwandeln, Speichern und Darstellen von Signal- bzw. Datenfolgen an oder in einen Recorder oder eine Recheneinheit (20), wobei die Recheneinheit (20) zur Durchführung des Verfahrens nach einem der Ansprüche 1 bis 6 ausgelegt ist.

8. Vorrichtung nach Anspruch 7, **gekennzeichnet durch** einen zusätzlich zu den bioakustischen Sensoren (12) vorgesehenen Lagesensor (17).

9. Vorrichtung nach Asnpruch 7 oder 8, **dadurch gekennzeichnet, dass** zusätzlich ein Sensor zum Abtasten von Brustkorbbewegungen vorgesehen ist, insbesondere zum Erfassen der Atemaktivität.

10. Vorrichtung nach einem der Ansprüche 7 bis 9, **dadurch gekennzeichnet, dass** die Komponenten in einem gemeinsamen Gehäuse, einen Koffer o.dgl. integriert sind.

11. Vorrichtung nach einem der Ansprüche 7 bis 10, **dadurch gekennzeichnet, dass** sie als am Körper tragbarer Datenlogger (10) ausgebildet ist, der einzeln, ambulant oder stationär verwendbar ist.

12. Vorrichtung nach Anspruch 11, **dadurch gekennzeichnet, dass** der Datenlogger (10) zur Überwachung von Körpergeräuschen und Aufzeichnung der Signale von maximal vier Geräuschkanälen mit akustischen Mikrospeichern ausgestattet ist, z.B. mit zwei Stereo-Mini-Einheiten in der Art von MP3-Recordern.

13. Vorrichtung nach Anspruch 11 oder 12, **dadurch gekennzeichnet, dass** der Datenlogger (10) mit einem Brustgurt (22) versehen ist, der an einem Objektkörper (K) anleg- und abnehmbar ist, zur Gewichtsentlastung Schulterträger (23) und insbesondere rückseitig zwei bioakustische Sensoren (14; 25) sowie vorderseitig eine Aufnahme (28) für eine Datenbox (29) aufweist, die über eine Zuleitung (30) mit einem Trachealmikrofon (31) verbunden ist.

## Claims

1. A method for detecting and monitoring body sounds in humans and animals using bioacoustic sensors and analyzers mounted downstream thereof, wherein bioacoustic sensors are fixed to maximally three selected points of an object body, maximally one additional bioacoustic sensor for recording surrounding noises is provided, sound signals and/or sound data are continuously recorded with the help of the sensors within a selectable time segment, the sequences of sound signals and/or sound data generated in this way are digitized, stored and transformed into frequency-dependent spectral curves, **characterized in that**, in a consecutively associated manner, at least two frequency ranges are selected in each case from the spectral curves, and medians of the amplitudes are determined which are provided as support values for adaption lines, associated adaption lines being developed by linear or quasilinear connection of the support-point values, and **in that** the adaption lines are compared with the corresponding spectral curves for detecting evaluable and signalizable specific sound symptoms, e.g. wheezing, ronchus, cough, or the like.

2. The method of claim 1, **characterized by** such a choice of the transformation time window that resolutions of approx. 0.1 s result.

3. The method of any of claims 1 or 2, **characterized in that** a Fourier Transformation or a Fast Fourier Transformation of the data is effected in a spectral range, wherein a spectral curve is generated per transformation time window, with which amplitudes can be plotted, transmitted and recorded over the frequency.

4. The method of any of claims 1 to 3, **characterized in that** the support values in the xy-coordinate system are in each case y-values in a frequency point X, and **in that** the evaluation is effected by comparing the y-values of the spectral curve and of the adaption line by means of threshold values.

5. The method for detecting and monitoring respiration sounds in humans and animals using bioacoustic sensors and analyzers mounted downstream thereof, in particular of any of claims 1 to 4, **characterized in that** plausibility criteria are used for the detection of wheezing by means of verified measuring results, namely:
i) the local maximum in the frequency range within a transformation time window is not wider than 100 Hz when the threshold value is exceeded;
ii) the maximums lie inside a frequency band between 150 Hz and 1600 Hz;
iii) the frequency change of the strongest one of the localen maximums (dominant frequency) is less than 100 Hz between two consecutive transformation time windows;
iv) the wheezing events have a minimum duration of 0.3 s, i.e. they occur in at least four consecutive transformation time windows when the temporal resolution is 0.1 s;
v) the wheezing events do not last longer than double the time of a normal mean expiration phase of the particular mammal species (i.e. in case of humans, < 2 x approx. 4 s).

6. The method for detecting and monitoring respiration sounds in humans and animals using bioacoustic sensors and analyzers mounted downstream thereof, in particular of any of claims 1 to 5, **characterized in that** the occurrence of steep flanks of the surrounding-noise amplitude signal is evaluated for cough detection, provided that at the same time, large amplitudes or overdrivings are detected in all other signals facing the object body.

7. An apparatus for detecting and monitoring body sounds in humans and animals using bioacoustic sensors (12) and analyzers mounted downstream thereof, to which sequences of data or signals are fed, in particular for carrying out the method of any of claims 1 to 6, **characterized by** the following components:
• maximally three bioacoustic sensors (12), each of which can be fixed to a point of an object body (K) facing the examination area,
• maximally one sensor (14) for recording surrounding noises,
• maximally four separable channels (11) for recording and transmitting sound signals or sound data detected by the sensors (12), and
• devices for supplying power and forwarding, converting, storing, and displaying sequences of signals or data to or in a recorder or a computer unit (20), the computer unit (20) being designed for carrying out the method of any of claims 1 to 6.

8. The apparatus of claim 7, **characterized by** a position sensor (17) provided in addition to the bioacoustic sensors (12).

9. The apparatus of claim 7 or 8, **characterized in that** a sensor for scanning movements of the thorax is additionally provided, in particular for detecting the respiration activity.

10. The apparatus of any of claims 7 to 9, **characterized in that** the components are integrated in a common housing, a case, or the like.

11. The apparatus of any of claims 7 to 10, **characterized in that** it is designed as a data logger (10) which can be carried on the body and which can be used individually, by in-patients or out-patients.

12. The apparatus of claim 11, **characterized in that** the data logger (10) is provided with acoustic micromemories, e.g. with two stereo mini units in the manner of MP3 recorders, for monitoring body sounds and recording the signals of maximally four sound channels.

13. The apparatus of claim 11 or 12, **characterized in that** the data logger (10) is provided with a breast belt (22), which can be applied on, and removed from, an object body (K), shoulder straps (23) for easing the weight, and in particular two bioacoustic sensors (14, 25) on the back as well as a receptacle (28) for a data box (29) which is connected to a tracheal microphone (31) via a supply cable (30).

## Revendications

1. Procédé pour détecter et surveiller des bruits de corps dans des humains et des animaux en utilisant des palpeurs bioacoustiques et des analyseurs placés en aval de ceux-ci, dans lequel des palpeurs bioacoustiques sont fixés à maximalement trois points sélectionnés d'un corps objet, maximalement un palpeur bioacoustique additionnel pour enregistrer des bruits ambiants est prévu, des signaux de bruit et/ou des données de bruit sont enregistrés continuellement à l'aide des palpeurs dans un segment de temps sélectionnable, les séquences de signaux de bruit et/ou de données de bruit générées de cette façon sont digitalisées, mémorisées et transformées en des courbes spectrales en fonction de la fréquence, **caractérisé en ce que**, d'une manière affectée consécutive, au moins deux gammes de fréquences sont sélectionnées à chaque fois à partir des courbes spectrales, et des médianes des amplitudes sont déterminées qui sont fournies comme valeurs de support pour des lignes d'adaptation, des lignes d'adaptation affectées étant développées par la connexion linéaire ou quasilinéaire des valeurs des points support, et **en ce que** les lignes d'adaptation sont comparées avec les courbes spectrales correspondantes pour détecter des symptômes de bruit spécifiques, par exemple wheezing, ronchus, toux, ou similaire.

2. Procédé selon la revendication 1, **caractérisé par** une telle sélection de la fenêtre de temps de transformation que des résolutions d'environ 0,1 s résultent.

3. Procédé selon l'une quelconque des revendications 1 ou 2, **caractérisé en ce que** une transformation Fourier ou une transformation Fourier rapide des données est effectuée dans un domaine spectral, dans lequel une courbe spectrale est générée par fenêtre de temps de transformation, avec laquelle des amplitudes peuvent être tracées, transmises et enregistrées sur la fréquence.

4. Procédé selon l'une quelconque des revendications 1 à 3, **caractérisé en ce que** les valeurs de support dans le système de coordonnées xy sont à chaque fois des valeurs y dans un point de fréquence X, et **en ce que** l'évaluation s'effectue par la comparaison des valeurs y de la courbe spectrale et de la ligne d'adaptation à l'aide de valeurs seuil.

5. Procédé pour détecter et surveiller des bruits de corps dans des humains et des animaux en utilisant des palpeurs bioacoustiques et des analyseurs placés en aval de ceux-ci, en particulier selon l'une quelconque des revendications 1 à 4, **caractérisé en ce que** des critères de plausibilité sont utilisés pour la détection de wheezing au moyen de résultats de mesurage vérifiés, à savoir:
i) le maximum local dans la gamme de fréquences à l'intérieur d'une fenêtre de temps de transformation n'est pas plus large que 100 Hz lorsque la valeur seuil est excédée;
ii) les maximums se trouvent à l'intérieur d'une bande de fréquence entre 150 Hz et 1600 Hz;
iii) le changement de fréquence du plus fort des maximums locaux (fréquence dominante) est moins de 100 Hz entre deux fenêtres de temps de transformation consécutives;
iv) les événements de wheezing ont une durée minimale de 0,3 s, c'est-à-dire qu'ils apparaissent dans au moins quatre fenêtres de temps de transformation consécutives lorsque la résolution temporelle est de 0,1 s;
v) les événements de wheezing ne durent plus long que le double du temps d'une phase normale d'expiration moyenne du genre particulier des mammifères (c'est-à-dire dans le cas des humains, < 2 x environ 4 s).

6. Procédé pour détecter et surveiller des bruits de corps dans des humains et des animaux en utilisant des palpeurs bioacoustiques et des analyseurs placés en aval de ceux-ci, en particulier selon l'une quelconque des revendications 1 à 5, **caractérisé en ce que** l'apparition de flancs raides du signal de l'amplitude du bruit ambiant est évaluée pour la détection de toux, pourvu que, au même temps, de grandes amplitudes ou surcharges sont détectées dans tous les autres signaux en regard du corps objet.

7. Dispositif pour détecter et surveiller des bruits dans des humains et des animaux en utilisant des palpeurs bioacoustiques (12) et des analyseurs placés en aval de ceux-ci, auxquels des séquences de données ou de signaux sont alimentées, en particulier pour exécuter le procédé selon l'une quelconque des revendications 1 à 6, **caractérisé par** les composants suivants:
• trois palpeurs bioacoustique (12) au maximum, dont chacun peut être fixé à un point d'un corps objet (K) en regard de la zone d'examen,
• un palpeur (14) au maximum pour enregistrer des bruits ambiants,
• quatre canaux (11) séparables pour enregistrer et transmettre des signaux de bruit ou des données de bruit détectés par les palpeurs (12) et
• des dispositifs pour alimenter de l'énergie et pour réacheminer, convertir, mémoriser et afficher des séquences de signaux ou de données à ou dans un enregistreur ou une unité d'ordinateur (20), l'unité d'ordinateur (20) étant conçue pour exécuter le procédé selon l'une quelconque des revendications 1 à 6.

8. Dispositif selon la revendication 7, **caractérisé par** un palpeur de position (17) prévu en plus des palpeurs bioacoustiques (12).

9. Dispositif selon la revendication 7 ou 8, **caractérisé en ce qu'**un palpeur pour balayer des mouvements du thorax est prévu additionellement, en particulier pour détecter l'activité de respiration.

10. Dispositif selon l'une quelconque des revendications 7 à 9, **caractérisé en ce que** les composants sont intégrés dans un boîtier commun, un coffre, ou similaire.

11. Dispositif selon l'une quelconque des revendications 7 à 10, **caractérisé en ce qu'**il est conçu comme un logger (10) de données qui peut être porté sur le corps et qui peut être utilisé de manière individuelle, par des patients hospitalisés ou des patients non hospitalisés.

12. Dispositif selon la revendication 11, **caractérisé en ce que** le logger (10) de données est muni de micromémoires acoustiques, c'est-à-dire de deux unités mini stéréo de la manière d'enregistreurs MP3, pour surveiller des bruits de corps et enregistrer les signaux de quatre canaux de bruit au maximum.

13. Dispositif selon la revendication 11 ou 12, **caractérisé en ce que** le logger (10) de données est muni d'une ceinture de poitrine (22) qui peut être mise sur un corps objet (K) et enlevée de celui-ci, des bretelles d'épaule (23) pour soulager le poids, et en particulier deux palpeurs bioacoustiques (14, 25) sur le dos ainsi qu'un logement (28) pour une boîte de données (29) qui est reliée à un microphone trachéal (31) à l'aide d'un câble d'alimentation (30).
